(19) Europäisches Patentamt
European Patent Office
Office européen des brevets

(11) Publication number: **0 347 459 A1**

(12) # EUROPEAN PATENT APPLICATION

published in accordance with Art. 158(3) EPC

(21) Application number: **88901320.7**

(22) Date of filing: **02.02.88**

Data of the international application taken as a basis:

(86) International application number: **PCT/JP88/00090**

(87) International publication number: **WO88/05777 (11.08.88 88/18)**

(51) Int. Cl.$^3$: **C 07 D 501/46**
C 07 D 501/57, A 61 K 31/54-5

(30) Priority: **03.02.87 JP 21852/87**

(43) Date of publication of application: **27.12.89 Bulletin 89/52**

(84) Designated Contracting States: **CH DE FR GB LI**

(71) Applicant: **TEIJIN LIMITED**
**11 Minamihonmachi 1-chome Higashi-ku**
**Osaka-shi Osaka 541(JP)**

(72) Inventor: **HARADA, Toshiaki**
**69-5, Ushinoya-machi 3-chome Iwakuni-shi**
**Yamaguchi 741(JP)**

(72) Inventor: **YOSHISATO, Eishin**
**110-22, Minamiiwakuni-machi 2-chome**
**Iwakuni-shi Yamaguchi 740(JP)**

(72) Inventor: **IMAI, Hiroshi**
**9-2, Yamate-cho 2-chome Iwakuni-shi**
**Yamaguchi 740(JP)**

(72) Inventor: **TAKANO, Yasunobu**
**9-2, yamate-cho 2-chome**
**Iwakuni-shi Yamaguchi 740(JP)**

(72) Inventor: **ICHIKAWA, Yataro**
**11-7, Kotesashi-machi 2-chome**
**Tokorozawa-shi Saitama 359(JP)**

(72) Inventor: **SUZUKI, Yoji**
**20-2, Tamadaira 5-chome**
**Hino-shi Tokyo 191(JP)**

(74) Representative: **Myerscough, Philip Boyd et al,**
**J.A.Kemp & Co. 14, South Square Gray's Inn**
**London, WC1R 5EU(GB)**

(54) **CEPHALOSPORIN COMPOUNDS OR THEIR SALTS, PROCESS FOR THEIR PREPARATION, AND PHARMACEUTICAL COMPOSITIONS.**

(57) Cephalosporin compounds represented by general formula (I)

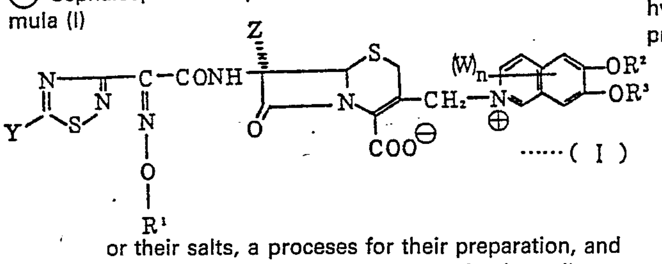

……( I )

or their salts, a processes for their preparation, and antibacterial agents containing them as active ingredients, wherein X represents −CH= or −N=; Y represents an optionally protected amino group; Z represents a hydrogen atom, a methoxy group or a formylamino group; $R^1$ represents an optionally substituted aromatic hydrocarbyl group; $R^2$ and $R^3$, which may be the same or different, each represents a hydrogen atom or a hydroxy group protecting group; W represents a substituent; and n represents an integer of 0 to 5.

EP 0 347 459 A1

SPECIFICATION

Cephalosporin compounds or their salts, process for production of same and their pharmaceutical compositions

## BACKGROUND OF INVENTION

### 1. Field of Invention

This invention relates to a novel cephalosporin compound or its salt, process for the production of same and its pharmaceutical composition. More specifically, this invention relates to a novel cephalosporin compound having a 6,7-dihydroxyisoquinoliniummethyl group in the 3-position or its pharmaceutically acceptable salt, a process for the production of same, and its pharmaceutical composition. The Cephalosporin compound and its pharmaceutically acceptable salt in this invention have very high antibacterial activity and strong activity capable of hindering growth of wide-ranging Gram-positives and Gram-negatives, especially showing excellent antibacterial activity to Gram-negatives, above all, Pseudomonas aeruginosa.

### 2. Description of Prior Art

Cephalosporin compounds having e.g. a 2-substituted imino-2-(5-amino-1,2,4-thiadiazol-3-yl)acetamide group in the 7-position of a cephalosporin skeleton are known as compounds having antibacterial properties, and varied compounds shown below have been already proposed, for example.

(i) Japanese Patent Publication No. 58353/1983 (British Patent No. 1536281)

The cephalosporin compounds described in this document are principally compounds having a 2-substituted imino-2-(5-amino-1,2,4-thiadiazol-3-yl)acetamide group in the 7-position and a substituted methyl group of a nucleophilic compound residue in the 3-position.

(ii) Japanese Laid-open Patent Application No. 57386/1983 (British Patent No. 4396619)

This document proposes cephalosporin compounds having a group typified by a 2-substituted imino-2-(5-amino-1,2,4-thiadiazol-3-yl)acetamide in the 7-position and an isoquinoliniummethyl group that may have a specific substituent in the 3-position.

(iii) Japanese Laid-open Patent Application No. 130294/1984 (European Patent No. 111935)

This document discloses cephalosporin compounds having a 2-substituted imino-2-(5-amino-1,2,4-thiadiazol-3-yl)acetamide group in the 7-position and a quinoliniummethyl group or an isoquinoliniummethyl group that may have a specific substituent in the 3-position.

3. Objects and Outline of Invention

It is an object of this invention to provide a novel cephalosporin compound having a 6,7-dihydroxyisoquinoliniummethyl group in the 3-position and a specific substituent in the 7-position.

Another object of this invention is to provide a novel cephalosporin compound having wide-ranging antibacterial spectrum and high antibacterial activity.

Still another object of this invention is to provide a novel cephalosporin compound having very high antibacterial activity to Gram-positives and Gram-negatives.

Yet another object of this invention is to provide a novel cephalosporin compound having very high antibacterial activity to Gram-negatives such as Pseudomonas aeruginosa, Salmonella typhi, Enterobacter, etc. in particular.

A further object of this invention is to provide a novel cephalosporin compound having excellent water-solubility and low toxicity.

A still further object of this invention is to provide a process for the production of a novel cephalosporin compound and its phamaceutical use.

The other objects and advantages of this invention will be made clear from the following description.

According to studies of this invention, it is found that these objects and advantages are achieved by the following cephalosporin compound.

Namely, this invention provides a cephalosporin compound represented by formula (I)

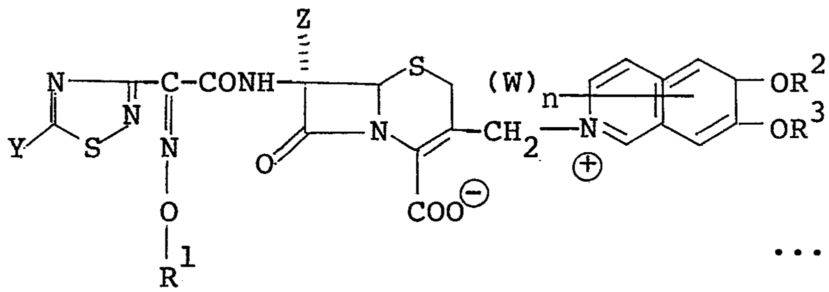

... (I)

wherein

Y denotes an amino group that may be protected,

Z denotes a hydrogen atom, a methoxy group or a formylamino group,

$R^1$ denotes an alkyl group that may be substituted or a hydrogen atom

$R^2$ and $R^3$ are the same or different, and each denotes a hydrogen atom or a protective group of a hydroxyl group,

W denotes a substituent, and

n is an integer of 0 to 5,

or its pharmaceutically acceptable salt.

One of the characteristics of such cephalosporin compound in this invention is that it has an isoquinoline skeleton of the following structure in the 3-position.

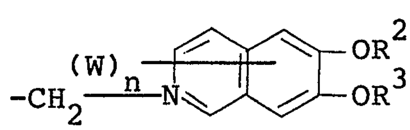

The isoquinoline skeleton has at least hydroxyl groups or protective groups thereof indicated by $-OR^2$ and $-OR^3$ in the 6- and 7-positions.

Another characteristic of the cephalosporin compound in this invention is that it has a thiadiazole group-containing acetamide group of the following structure in the 7-position.

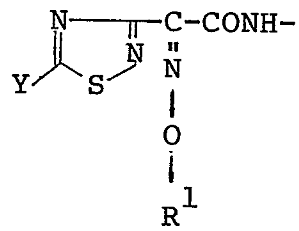

The acetamide group is characterized in that it has an imino group wherein $R^1$ is an alkyl group that may be substituted or a hydrogen atom, and further a 5-amino (or protected amino)-1,2,4-thiadiazol-3-yl group.

The cephalosporin compound of this invention having the above characteristics has such excellent physiological activity that it possesses wide-ranging antibacterial spectrum and very high antibacterial activity. Especially, it possesses wide-ranging antibacterial spectrum and very high antibacterial activity to Gram-negatives, and can hinder growth of bacteria in a surprisingly small amount. Particularly noteworthy is that the cephalosporin compound shows a higher activity to Pseudomonas aeruginosa than hitherto attainable with the commercial cephalosporin compounds.

4. Description of Preferable embodiments of Invention

The cephalosporin compound (I) of this invention is explained in more detail below.

The cephalosporin compound represented by formula (I) in this invention has partially a 5-amino-1,2,4-thiadiazol-3-yl skeleton reprsented by formula:

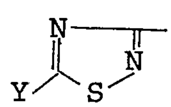

This skeleton can easily be isomerized to an amino-thiadiazoline skeleton represented by formula:

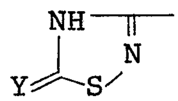

In this invention, either interchangeable isomer or a mixture of these isomers will do.

Moreover, the cephalosporin compound of formula I in this invention has partially an imino group represented by formula:

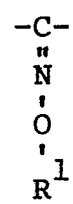

The imino group moiety to which $R^1$-O- is bound includes a syn-form and an anti-form. Of these, the syn-form is preferable becuase of the higher antibacterial activity.

Y in formula (I) is an amino group that may be protected, and therefore means an amino group itself or an amino group protected with a protective group. In case of the protected amino group, the protective group is preferably an ordinary protective group of an amino group. Examples of the protective group are an acyl group that may be substituted, an aralkyl group that may be substituted and a silyl group that may be substituted. Examples of such acyl group are lower alkanoyl groups such as formyl, acetyl, propionyl, butyryl, iso-butyryl, valeryl, iso-valeryl, oxalyl, succinyl, pivaloyl and trityloxyacetyl; lower alkoxycarbonyl groups such as methoxycarbonyl, ethoxycarbonyl, propoxycarbonyl, isopropoxycarbonyl, butoxycarbonyl and pentyloxycarbonyl; lower alkanesulfonyl groups such as mesyl, ethanesulfonyl,

propanesulfonyl, iso-propanesulfonyl and butanesulfonyl; aroyl groups such as benzoyl, toluoyl, naphthoyl and phthaloyl; aralkanoyl groups such as phenylacetyl and phenylpropionyl; and aralkoxycarbonyl groups such as benzoyloxycarbonyl. Such acyl group may have one or more suitable substituents. Examples of the substituent are a halogen atom such as chlorine, bromine, iodine or fluorine, a cyano group, and a lower alkyl group such as methyl, ethyl, propyl or butyl. Examples of the aralkyl group that may have a substituent are benzyl, 4-methoxybenzyl, phenetyl, trityl, and 3,4-dimethoxybenzyl.

Examples of the silyl group that may be substituted are trimethylsilyl, dimethyl-t-butylsilyl, methyl-di-t-butylsilyl, triphenylsilyl and diphenyl-t-butylsilyl.

$R^1$ in formula (I) of the sephalosporin compound in this invention is a hydrogen atom or an alkyl group that may be substituted. On this occasion, examples of the unsubstituted alkyl group are linear or branched $C_1$-$C_{10}$ alkyl groups such as methyl, ethyl, n-propyl, iso-propyl, n-butyl, sec-butyl, tert-butyl, pentyl, hexyl, heptyl, octyl, nonyl and decyl. When such alkyl group has a substituent, examples of the substituent are a carboxy group; an alkoxycarbonyl group such as methoxycarbonyl, ethoxycarbonyl, tert-butoxycarbonyl or pentyloxycarbonyl; and a heterocyclic group such as imidazolyl, 5-methylimidazolyl, 1-methylimidazolyl, 2-methyl-5-nitro-3-imidazolyl, 2-aminothiazolyl, 1H-tetrazolyl, 2H-tetrazolyl, thiazolyl, 1-methyl-1H-tetrazolyl or pyrrolidon-3-yl.

Preferable examples of $R^1$ are lower alkyl groups such as methyl, ethyl, propyl, iso-propyl, butyl, tert-butyl and hexyl; and lower alkyl groups substituted with a carboxyl group, such as carboxymethyl, 2-carboxyethyl, 2-carboxy-2-butyl and 3-carboxy-3-pentyl.

Z in formula (I) is a hydrogen atom, a methoxy group or a formylamide group. Of these, the hydrogen

atom and the methoxy group are preferable, and the hydrogen atom is most preferable.

As noted above, the cephalosporin compound in this invention has, in the 3-position, an isoquinoline skeleton having at least groups indicated by $-OR^2$ and $-OR^2$ in the 6- and 7-positions as shown in formula (I). $R^2$ and $R^3$ are, independently from each other, a hydrogen atom or a protective group of a hydroxyl group. The protective group of the hydroxyl group is a group convertible into a hydroxyl group chemically or biolocally. $R^2$ and $R^3$ may together form a ring with an oxygen atom to which they are bound. An acyl group with 2 to 4 carbon atoms is preferable. The hydrogen atom of the acyl group may have further a substituent such as a hydroxyl group, an amino group or a carboxyl group. Examples of the silyl group are trialkylsilyl groups such as a trimethylsilyl group, a triethylsilyl group, a tert-butyldimethylsilyl group, a di-tert-butylmethylsilyl group and a tributylsilyl groups; and dialkylsilyl groups wherein $R^2$ and $R^3$ together form a ring, such as a di-tert-butylsilyl group, a diethylbutylsilyl group and a di(di-tert-butylsiloxy) group.

The phosphoric acid group or the sulfuric acid group is a group forming a salt or an esteramide. Examples thereof are $-OPO(OH)_2$, $-OPO(OH)(ONa)$, $-OPO(OH)(OK)$, $-OPO(OKa)_2$, $-OPO(OK)_2$, $-OPO(OH)(OR^6)$, $-OPO(OR^6)_2$, $OPO(OR^6)(OR^7)$, $-OPO(OH)(N\text{-}H_2)$, $-OPO(OH)(NHR^6)$,

$$-OPO(N\begin{smallmatrix}R^6\\ R^7\end{smallmatrix})_2,\ -OSO_2(OH),\ -OSO_2(OK),\ OSO_2(ONa),\ OSO_2NH_2,$$

$OSO_2\text{-}(NR^6R^7)$, and $OSO_2R^6$. In these examples of the phosphoric acid group or the sulfuric acid group, $R^6$ and $R^7$ may be the same or different, and each denotes an alkyl group with 1 to 4 carbon atoms.

Moreover, examples of $R^2$ and $R^3$ are $-OPO(R^9)O-$, $-OPO(OR^8)O-$, $OSO_2O-$, $-OCOO-$, $-OSi(R^9)_2O-$,

$-OSi(R^9)_2-O-Si(R^9)-O-$, $OPO(OR^8)O(OR^8)OPO-$ and $OP-(OH)O-$. Where $R^2$ and $R^3$ form part of a ring, $R^8$ is a hydrogen atom, a metal ion, an ammonium ion that may be substituted, an alkyl group that may be substituted or an aryl group that may be substituted. The phosphonic acid group is a group represented by formula -O-PO-(A)(OB) wherein A is an alkyl group that may be substituted or an aryl group that may be substituted, and B is a hydrogen atom, a metal ion, an ammonium ion that may be substituted, an alkyl group that may be substituted or an aryl group that may be substituted. Or it may form a ring with an adjacent hydrogen group to give -O-OP(A)-O-. The sulfonic acid group is one represented by formula $-O-S(O)_2A$ wherein A is an alkyl group or an aryl group that may be substituted.

The isoquinoline skeleton may have two substituents $-OR^2$ and $-OR^3$ in the 6- and 7-positions or further the other substituent $[(W)_n]$ in the other position. In this case, the number (n) of substituents is 0 to 5, preferably 0 to 2, and most preferably 0 to 1. Examples of the substituent (-W) may be varied substituents; it can be the same as those of $R^1$ shown above.

Examples of the substituent (-W) are an amino group, an alkylamino group with 1 to 4 carbon atoms, a di(alkyl with 1 to 4 carbon atoms)amino group, a hydroxyl group, an alkoxy group with 1 to 4 carbon atoms, a carbamoyl group, a carbamoyloxy group, a thiocarbamoyl group, an acyloxy group with 2 to 4 carbon atoms, a cyano gorup, a halogen atom, a halogenated alkyl group with 1 to 4 carbon atoms, a sulfo group, an aminosulfonyl group, an alkanoyl group with 2 to 4 carbon atoms, an alkyl group with 1 to 4 carbon atoms, a carboxyl group, an alkoxycarbonyl group with 1 to 4 carbon atoms, a hydroxyl group, an alkyl group with 1 to 4 carbon atom, and a formyl group.

In the cephalosporin compound of formula (I) in

this invention, a cation+ in the 3-position forms salts with varied anions. Examples of the anions are ions of monobasic acids such as $Cl^-$, $Br^-$, $HCOO^-$, $-CH_3COO^-$, $CCl_3COO^-$ and $CF_3COO^-$.

Other salts of the cephalosporin compound represented by formula (I) are salts of said compound having a carboxyl group ($-COO^-$) in the 4-position. Examples of the salts are alkali metal salts such as sodium and potassium salts, alkaline earth metal salts such as calcium and magnesium salts, ammonium salts, salts with organic bases such as triethylammonium, trimethylammonium, pyridinium, tetraethylammonium, tetra-n-butylammonium salts.

The cephalosporin compound of formula (I) may be an ester with the carboxyl group in the 4-position esterified. Such ester may be an ester that is easily hydrolyzed physiologically. Examples of such ester are alkoxyalkyl esters such as methoxymethyl, ethoxymethyl, methoxyethyl, ethoxyethyl esters; alkanoyloxyalkyl esters such as acetoxymethyl, propioxymethyl, butyryloxymethyl, valeryloxymethyl and pivaloyloxymethyl esters; and indanyl, phthalidyl, glycyloxymethyl and phenylglycyloxymethyl esters.

In this invention, the cephalosporin compound, its salt or its ester in crystalline form is chemically stable. Therefore, the cephalosporin compound, its salt or its ester in crystalline form is desirous when used as an active ingredient of a pharmaceutical composition. The crystals may be anhydrides or hydrates such as a 3/4-hydrate, a 1-hydrate, a 1,5-hydrate, a 3-hydrate and a 5-hydrate.

Preferable concrete examples of the cephalosporin compound in this invention are as follows. This invention is however limited to them.

(1) (6R,7R)-7-[(Z)-2-(5-amino-1,2,4-thiadiazol-3-yl)-2-methoxyiminoacetamide]-3-(6,7-dihydroxy-2-isoquinolinium)methyl-3-cephem-4-carboxylate

(2) (6R,7R)-7-[(Z)-2-(5-amino-1,2,4-thiadiazol-3-yl)-2-ethoxyiminoacetamide]-3-(6,7-dihydroxy-2-isoquinolinium)methyl-3-cephem-4-carboxylate

(3) (6R,7R)-7-[(Z)-2-(5-amino-1,2,4-thiadiazol-3-yl)-2-carboxymethoxyiminoacetamide]-3-(6,7-dihydroxy-2-isoquinolinium)methyl-3-cephem-4-carboxylate

(4) (6R,7R)-7-[(Z)-2-(5-amino-1,2,4-thiadiazol-3-yl)-2-(2-carboxypropyl-2-yloxyimino)acetamide]-3-(6,7-dihydroxy-2-isoquinolinium)methyl-3-cephem-4-carboxylate

(5) (6R,7R)-7-[(Z)-2-(5-amino-1,2,4-thiadiazol-3-yl)-2-aryloxyiminoacetamide]-3-(6,7-dihydroxy-2-isoquinolinium)methyl-3-cephem-4-carboxylate

(6) (6R,7R)-7-[(Z)-2-(5-amino-1,2,4-thiadiazol-3-yl)-2-(phenylthio)methoxyiminoacetamide]-3-(6,7-dihydroxy-2-isoquinolinium)methyl-3-cephem-4-carboxylate

(7) (6R,7R)-7-[(Z)-2-(5-amino-1,2,4-thiadiazol-3-yl)-2-(1-methyltetrazol-5-thioyl)methoxyiminoacetamide]-3-(6,7-dihydroxy-2-isoquinolinium)methyl-3-cephem-4-carboxylate

(8) (6R,7R)-7-[(Z)-2-(5-amino-1,2,4-thiadiazol-3-yl)-2-(5-methylimidazol-4-ylmethoxyimino)acetamide]-3-(6,7-dihydroxy-2-isoquinolinium)methyl-2-cephem-4-carboxylate

(9) (6R,7R)-7-[(Z)-2-(5-amino-1,2,4-thiadiazol-3-yl)-2-(2-carboxypropyl-2-thioylmethoxyiminoacetamide]-3-(6,7-dihydroxy-2-isoquinolinium)methyl-3-cephem-4-carboxylate

(10) (6R,7R)-7-[(Z)-2-(5-amino-1,2,4-thiadiazol-3-yl)-2-methoxyiminoacetamide]-3-(4-carboxy-6,7-dihydroxy-2-isoquinolinium)methyl-3-cephem-4-carboxylate

(11) (6R,7R)-7-[(Z)-2-(5-amino-1,2,4-thiadiazol-3-yl)-2-(chloromethylthiomethyloxyimino)acetamide]-3-(6,7-dihydroxy-2-isoquinolinium)methyl-3-cephem-4-carboxylate

(12) (6R,7R)-7-[(Z)-2-(5-amino-1,2,4-thiadiazol-4-yl)-2-(5-methylimidazol-4-ylmethoxy)acetamide]-3-(6,7-dihydroxy-2-isoquinolinium)methyl-3-cephem-4-carboxylate

(13) (6R,7R)-7-[(Z)-2-(5-amino-1,2,4-thiadiazol-3-yl)-2-(2-pyrolidon-3-yloxyimino)acetamide]-3-(6,7-dihydroxy-2-isoquinolinium)methyl-3-cephem-4-carboxylate

(14) (6R,7R)-7-[(Z)-2-(5-amino-1,2,4-thiadiazol-3-yl)-2-(2,2,2-trifluoroethoxyimino)acetamide]-3-(6,7-dihydroxy-2-isoquinolinium)methyl-3-cephem-4-carboxylate

(15) (6R,7R)-7-[(Z)-2-(5-amino-1,2,4-thiadiazol-3-yl-2-hydroxyimino)acetamide]-3-(6,7-dihydroxy-2-isoquinolinium)methyl-3-cephem-4-carboxylate

(16) (6R,7R)-7-[(Z)-2-(5-amino-1,2,4-thiadiazol-3-yl-2-(2-carboxypropyl-2-yloxyimino)acetamide]-3-(6,7-dioxy-di-tert-butylsilyl-2-isoquinolinium)methyl-3-cephem-4-carboxylate

The cephalosporin compound of formula (I), its salt or its ester in this invention can be obtained by varied synthesis methods. Several of them are explained below. The cephalosporin compound in this invention is however not limited by these synthesis compounds.

<u>Reaction scheme A</u>

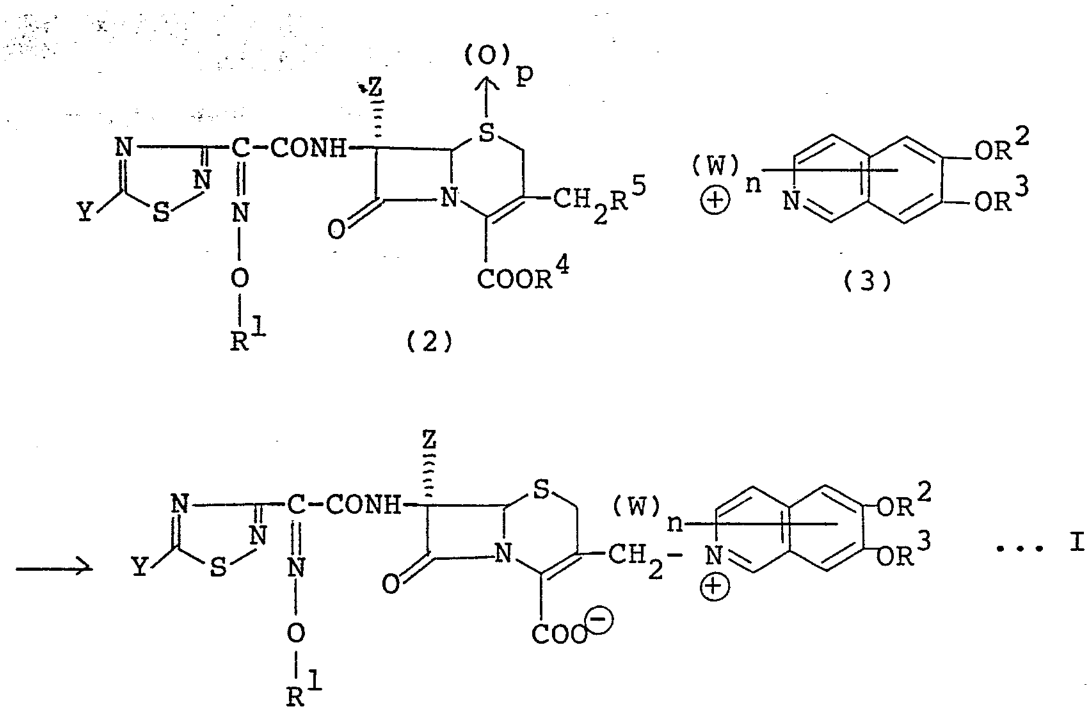

(2)

(3)

... I

In the reaction scheme A, the cephalosporin compound of formula (2) is reacted with the isoquinoline compound of formula (3). The reaction product may be subjected to deprotection, salt formation, esterification and/or reduction if required.

In formula (2), Y, Z and $R^1$ are as defined in formula (1), $R^5$ denotes an acyloxy group, a carbamoyloxy group or a halogen atom, p is 0 or 1 and $R^4$ denotes a hydrogen atom or a protective group.

Preferable examples of the acyloxy group in $R^5$ are acetyloxy, trifluoroacetyloxy, trichloroacetyloxy, propionyloxy, 3-oxobutyloxy, 3-carboxypropionyloxy, 2-carboxybenzoyloxy, 4-carboxybutylyloxy, mandelyloxy, 2-(carboethoxycarbamoyl)benzoyloxy, 2-(carboethoxysulfamoyl)benzoyloxy and 3-ethoxycarbamoylpropionyloxy. Examples of the halogen atom are iodine, bromine and chlorine.

Examples of the protective group in $R^4$ are lower alkyl groups such as methyl, ethyl, propyl, isopropyl, butyl, pentyl and hexyl; alkanoyloxyalkyl groups such as acetoxymethyl, propionyloxymethyl, butyryloxymethyl, valeryloxymethyl and pivaloyloxymethyl; alkoxyalkyl groups such as methoxymethyl, ethoxymethyl, methoxyethyl and ethoxyethyl; aralkyl groups that may be substituted, such as benzyl, 4-methoxybenzyl, 4-nitrobenzyl, phenethyl, trityl, diphenylmethyl, bis(methoxyphenyl)methyl and 3,4-dimethoxybenzyl; halogenated alkyl groups such as 2-iodoethyl and 2,2,2-trichloroethyl; alkenyl groups such as vinyl and allyl; aryl groups that may be substituted, such as phenyl, 4-chlorophenyl, tolyl, xylyl and mesityl; and trialkylsilyl groups such as trimethylsilyl, triethylsilyl, tert-butyldimethylsilyl, and tributylsilyl. Above all, the trialkylsilyl groups are preferable as $R^4$. When $R^4$ is the trialkylsilyl groups, formation of a $\Delta^2$-isomer of the cephalosporin derivative represented by formula (1) is

suppresed in the reaction scheme A, whereby the intended cephalosporin compound of formula (1) is obtained in high yield.

The cephalosporin compound of formula (2) may be in salt form. Preferable examples of the salt can be the same salts as shown in the cephalosporin compound of formula (1).

W and n in formula (3) are as defined in formula (1).

The cephalosporin compound of formula (3) may be in salt form. Preferable examples of the salt are inorganic salts such as a hydrochloride, a hydrobromide, a sulfate and a phosphate; and organic acid salts such as an acetate, a maleate, a tartarate, a benzenesulfonate and a toluenesulfonate.

The reaction between the cephalosporin compound of formula (2) or its salt and the isoquinoline compound of formula (3) or its salt is carried out by contacting them in an inactive organic solvent.

Examples of the inactive organic solvent are halogenated hydrocarbons such as methylene chloride, dichloromethane, chloroform, carbon tetrachloride and 1,2-dichloroethane, ethers such as diethylether, tetrahydrofuran, dioxane and dimethoxyethane, hydrocarbons such as hexane, benzene, toluene and xylene, acetonitrile, dimethylformamide, diethylacetamide, dimethylsulfoxide, and ethyl acetate.

The reaction temperature is preferably room temperature or lower. It is advisable to perform the reaction usually in the temparature range of -30°C to +50°C.

The cephalosporin compound of formula (2) or its salt and the isoquinoline compound of formula (3) or its salt are reacted in equimolar amounts. In the reaction, the isoquinoline compound of formula (3) or its salt is used in an amount of 0.7 to 10 mols, preferably

1.0 to 10 mols per mol of the cephalosporin compound of formula (2) or its salt.

The reaction proceeds only by stirring both of them. The reaction time varies with reaction solvent and reaction temperature. It is usually 5 minutes to 3 hours. The reaction product can be isolated and purified by known means such as solvent extraction, crystallization and chromatography.

When in the reaction scheme A a syn-isomer of the compound of formula (2) is used as a starting compound, a syn-isomer of the end compound (1) is obtained. When a mixture of syn- and anti-isomers of the compound of formula (2) is used as a starting compound, a mixture of syn- and anti-isomers of the end compound (1) is obtained. The mixture can be separated by usual means such as crystallization and chromatography.

The reaction product is subjected to deprotection, salt formation, esterification and/or reduction if required. The deprotection reaction is a reaction known per se, and the protective group of the carboxyl group can be removed by a hydrolysis reaction in the presence of an acid or an alkali. The protective group of the amino group can be removed by acid or catalytic reduction (refer to U.S. Patent Nos. 4,152,433 and 4,298,606).

The salt formation reaction is a reaction known per se. For example, it is carried out by treating the compound of a betaine structure of formula (1) obtained by the above reaction with an acid such as hydrochloric acid, hydrobromic acid, sulfuric acid, phosphoric acid, acetic acid or trifluoroacetic acid or with a salt such as sodium chloride, sodium iodide or potassium chloride in the presence of an acid.

The esterification reaction is a reaction known per se, and it is carried out by reacting the resulting cephalosporin compound of formula (1) or its salt with an

alkanoyloxyalkyl halide or an alkoxyalkyl halide in an inactive organic solvent such as acetone or dimethylformamide (refer to British Patent No. 240921).

The reduction reaction is a reaction known per se, and it is carried out by treating the cephalosporin compound of formula (1) wherein p is 1 with acetyl chloride, and reducing the treated compound with an iodine ion or sodium dithionite (refer to British Patent No. 240921).

The cephalosporin compound of this invention in crystalline form is obtained by the following methods.

A method wherein the amorphous powder of the cephalosporin compound of formula (1) is dissolved in a solvent such as water or an organic solvent, for example, methanol, ethanol, acetone, tetrahydrofuran, dioxane or acetonitrile, or a mixture of them, left to stand and precipitated as a crystal; a method wherein the amorphous powder is recrystallized using these solvents; or a method wherein the amorphous powder is dissolved in water and then precipitated as a crystal with the addition of the organic solvent.

There is another method wherein an acid addition salt of the cephalosporin compound of formula (1) is dissolved in a solvent such as water or an organic solvent, for example, methanol, ethanol or propanol, or a solvent mixture of them, and then neutralized with a base such as triethylamine or sodium hydroxide to obtain a crystal.

When crystallization or recrystallization is carried out, as aforesaid, in water or an aqueous solution, crystalline hydrates are usually obtained. When crystallization is carried out in an organic solvent, crystalline anhydrides or solvates are obtained. The solvates are easily converted into hydrates. The crystalline anhydrides or solvates can be converted into hydrates by allowing them to stand in a steam-containing gas.

Reaction scheme B

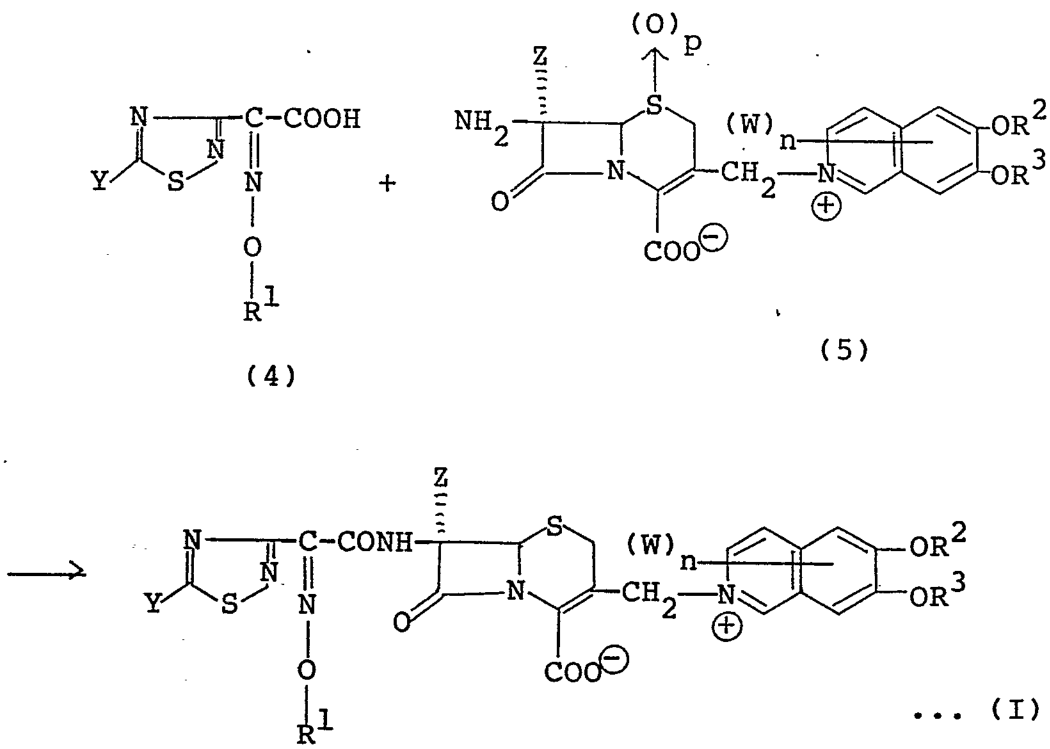

(4) (5)

... (I)

In the reaction scheme B, the compound of formula (4), its salt or its reactive derivative is reacted with the compound of formula (5), its salt, its ester or its reactive derivative. The reaction product is subjected to deprotection, salt formation, esterification and/or reduction if required.

In formula (4), Y and $R^1$ are as defined in formula (1).

The compound of formula (4) may be in salt form, and the salt is an acid addition salt. Examples of such acid addition salt can be the same as the acid addition salts of the compound of formula (3) in the reaction scheme A.

Examples of the reactive derivative of the compound of formula (4) are an acid halide, an acid anhydride, a mixed acid anhydride activated amide and an activated ester.

Examples of the acid halide are an acid chloride and an acid bromide. Examples of the mixed acid anhydride are anhydrides of mixtures of acids such as a dialkylphosphoric acid, phenylphosphoric acid, pivalic acid and pentanoic acid. Examples of the activated amide are amides activated with imidazole, triazole, tetrazole and dimethylpyrazole. Examples of the activated ester are cyanomethyl ester, methoxymethyl ester, dimethyliminomethyl ester, p-nitrophenyl ester and mesylphenyl ester.

In formula (5), Z, $R^2$, $R^3$, W and n are as defined in formula (1). Examples of the salt of the compound of formula (5) are alkali metal salts such as a sodium salt and a potassium salt; alkaline earth metal salts such as a calcium salt, and a magnesium salt; an ammonium salt; salts with organic bases such as a trimethylammonium salt, a triethylammonium salt, a pyridinium salt, a tetraethylammonium salt and a tetra-n-butylammonium salt; and the same acid addition salts as snown above.

Examples of the ester of the compound of formula (5) can be compounds esterified with the protective group in $R^2$ of the compound of formula (2) in the reaction scheme A.

Examples of the reactive derivative of the compound of formula (5) are Schiff base-type imino compounds formed by the reaction with a carbonyl compound such as acetoacetic acid or their enamine-type isomers; silyl derivatives formed by the reaction with silyl compounds such as bis(trimethylsilyl)acetamide, trimethylchlorosilane and tert-butyldimethylchlorosilane; and derivatives obtained by the reaction with phosphorus trichloride and phosgene.

The compound of formula (5) can be produced by the following method.

Synthetic method of the compound of formula (5)

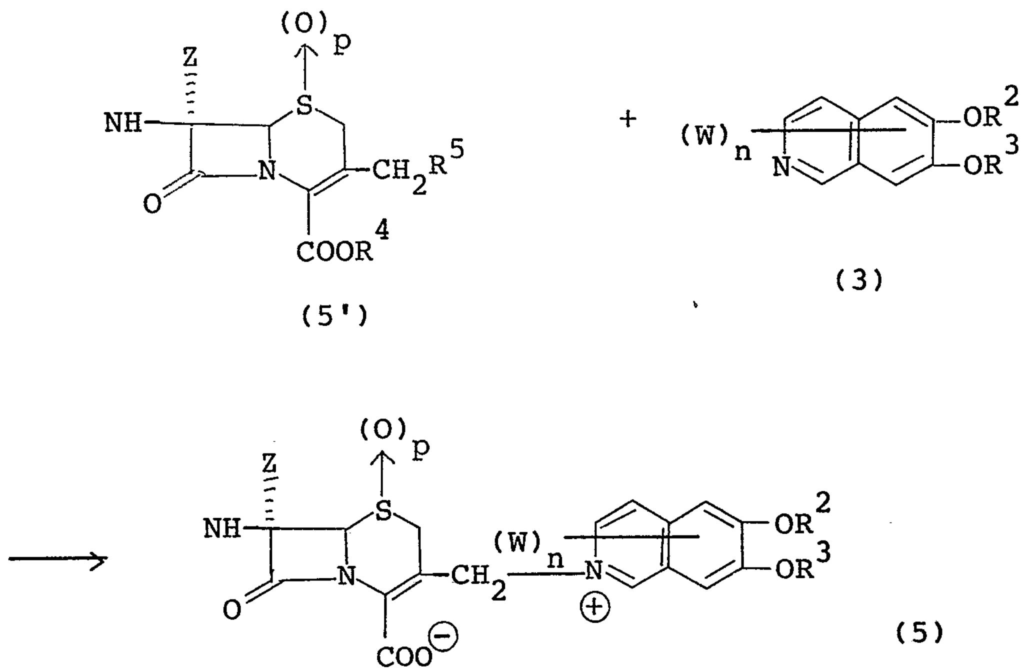

(5') (3) (5)

The above reaction can be carried out in the same way as the reaction between the compound of formula (2) and the isoquinoline compound of formula (3) by the reaction scheme A.

The reaction of the compound of formula (4), its salt or its reactive derivative with the compound of formula (5), its salt, its ester or its reactive derivative is performed by contacting both of them in an inactive organic solvent and if required, in the presence of a condensation agent or a base.

Examples of the inactive organic solvent are halogenated hydrocarbons such as methylene chloride, dichloromethane, chloroform, carbon tetrachloride and 1,2-dichloroethane, ethers such as diethyl ether, tetrahydrofuran, dioxane and dimethoxyethane, hydrocarbons such as hexane, benzene, toluene and xylene, acetonitrile, dimethylformamide and diethyl sulfoxide.

In the reaction, a base may be added. Examples of the base are inorganic bases such as an alkali metal

hydroxide, an alkali metal bicarbonate and an alkali metal carbonate; and organic bases such as a trialkylamine, a N,N-dialkylbenzylamine, pyridine and N-alkylmorpholine. The amount of such base is usually 1 to 3 mols per mol of the compound of formula (4), its salt, or its reactive derivative.

When the compound of formula (4) or its salt is used in the reaction, the condensation agent may be added. Examples of the condensation agent are N,N'-dicyclohexylcarbodiimide, N-cyclohexyl-N'-morpholinoethylcarbodiimide, N,N'-diethylcarbodiimide, trimethylphosphite, triphenylphosphine, and 2-ethyl-7-hydroxybenzisoxazolium salt. The amount of such condensation agent is 1 to 3 mols per mol of the compound of formula (4) or its salt.

The compound of formula (4), its salt or its reactive derivative and the compound of formula (5), its salt or its reactive derivative are reacted usually in approximately equimolar amounts.

The reaction is usually performed under cooling or at room temperature. The reaction is usually finished in several minutes to several tens of hours. The reaction product can be isolated and purified by known means such as solvent extraction, crystallization and chromatography.

The deprotection, salt formation, esterification and/ or reduction of the reaction product can be carried out in the same way as shown in the reaction scheme A. Even the crystal of the end compound can be afforded in the same manner as shown in the reaction scheme A.

When a syn-isomer of the compound of formula (4) is used as a starting compound in this reaction, a syn-isomer of the end compound of formula (1) is obtained. When a mixture of syn- and anti-isomers of the compound of formula (4) is used as a starting compound, a

mixture of syn- and anti-isomers of the end compound of formula (1) is obtained. Such mixture can be separated by usual means such as crystallization and chromatography.

Reaction scheme C

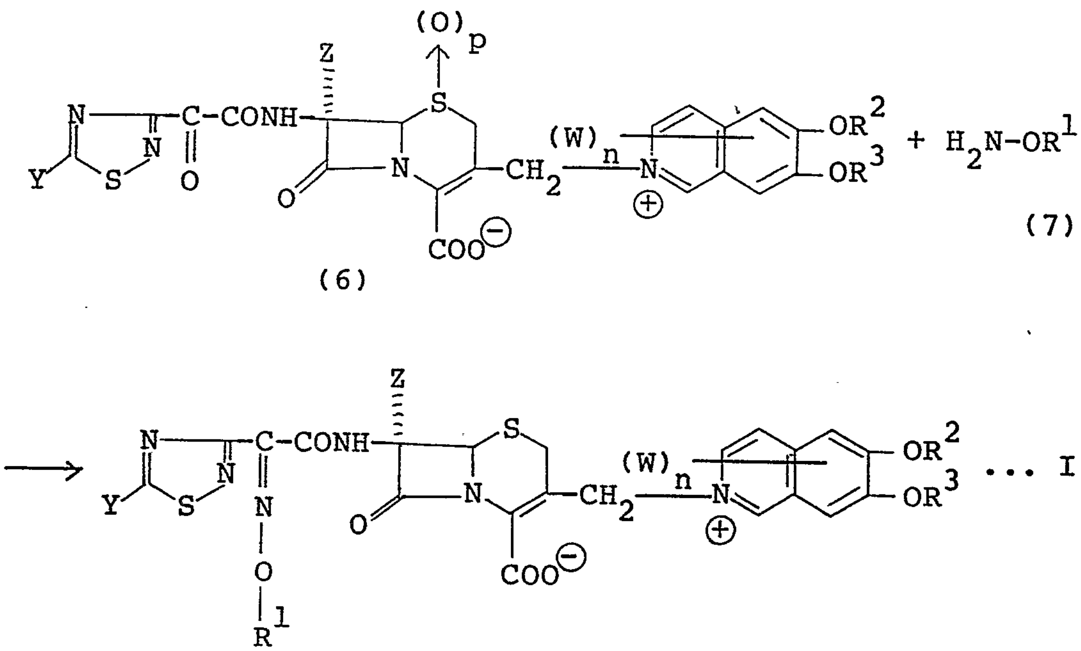

(6) (7) ... I

In the reaction scheme C, the compound of formula (6), its salt or its ester is reacted with the compound of formula (7), its protected compound or its salt. The reaction product is subjected to deprotection, salt formation, esterification and/or reduction if required.

In formula (6), Y, Z, $R^2$, $R^3$, W, n and p are as defined above.

Examples of the salt of the compound of formula (6) are an acid addition salt, an alkali metal salt, an alkaline earth metal salt, an ammonium salt and a salt with an organic base. Concrete Examples of these salts can be the same salts of the compound of formula (1).

The ester of the compound of formula (6) can be, for example, a compound esterified with the group

shown as the protective group in $R^4$ of the compound of formula (2) in the reaction scheme A.

The compound of formula (6) can be formed by the following method.

<u>Synthetic method of the compound of formula (6)</u>

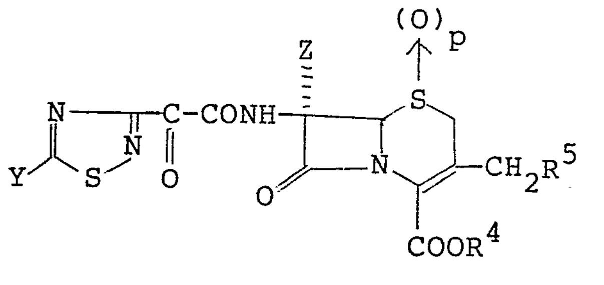

(6')

+

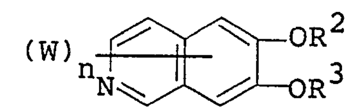

(3)

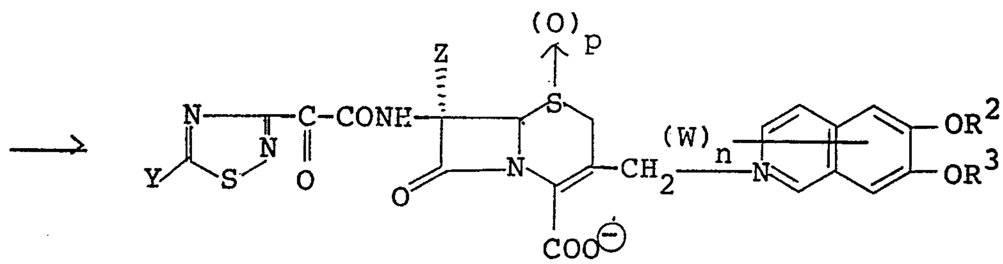

(6)

The above reaction can be performed in the same way as the reaction between the compound of formula (2) and the isoquinoline compound of formula (3) by the reaction scheme A.

In formula (7), $R^1$ is as defined above. Regarding the protected one of the compound of formula (7), when $R^1$ has an active substituent such as a carboxyl group, a compound with the substituent protected by an ordinary protective group can be taken.

The salt of the compound of formula (7) is an acid addition salt and concrete examples thereof can be the same as indicated above.

The compound of formula (7) is a known compound and can be formed by a known method using hydroxyphthalimide (Bulletin Society, 833, 1976) or a method

using 3,4-dinitrophenolhydroxyamine (Japanese Laid-open Patent Application No. 169446/ 1985).

The reaction of the compound of formula (6), its salt or its ester with the compound of formula (7), its protected compound or its salt is carried out by contacting them in an inactive organic solvent and if required, in the presence of a base.

Examples of the inactive organic solvent are alcohols such as methanol, ethanol, propanol and butanol. These inactive organic solvents may contain water.

Examples of the base which is added if required can be the same as the examples of the base used in the reaction by the reaction scheme B.

The amount of such base is usually 1 to 3 mols per mol of the compound of formula (6), its salt or its ester. The compound of formula (6), its salt or its ester and the compound of formula (6), its protected compound or its salt are reacted in equimolar amounts.

The reaction is usually carried out under cooling or at room tempersture. The reaction time is usually several minutes to several tens of hours. The reaction product can be isolated and purified by known means such as solvent extraction, crystallization and chromatography. In this reaction, the mixture of the syn- and anti-isomers of the compound is obtained.

Such mixture can be separated by usual means such as crystallization and chromatography.

Deprotection, salt formation, esterification and/or reduction can be conducted in the same way as in the reaction scheme A. The crystalline anhydride or hydrate of the end compound can be also btained in the same way as in the reaction scheme A.

Reaction scheme D

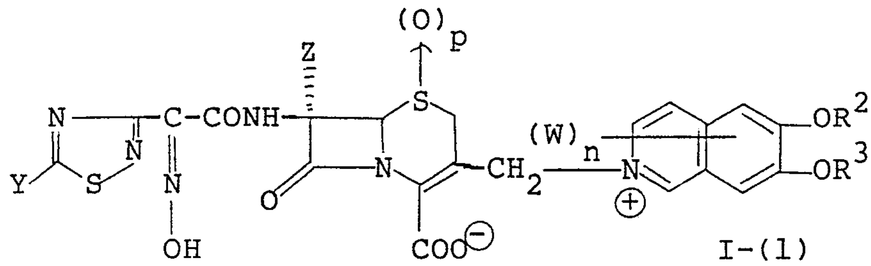

I-(1)

+ $Ha\ell - R^1$

(10)

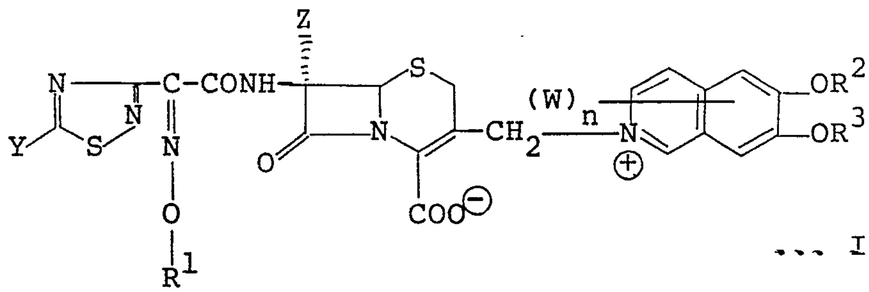

... I

In the reaction scheme D, the compound of formula I-(1), its salt or its ester is reacted with the compound of formula (10) in the presence of a dialkyl sulfate or a diazoalkane. The reaction product is subjected to deprotection, salt formation, esterification and/or reduction.

In formula I-(1), Y, Z, $R^1$, $R^2$, W, n and p are as defined above.

Examples of the salt of the compound of formula I-(1) are an acid addition salt, an alkali metal salt, an alkaline earth metal salt, an ammonium salt and a salt with an organic base. Concrete examples of these salts can be the same salts shown in the compound of formula (1).

The ester of the compound of formula I-(1) can be a compound esterified with the protective group in $R^4$ of the compound of formula (2) in the reaction scheme A.

The compound of formula I-(1) can be formed by the following method.

Synthetic method of the compound of formulaI-(1)

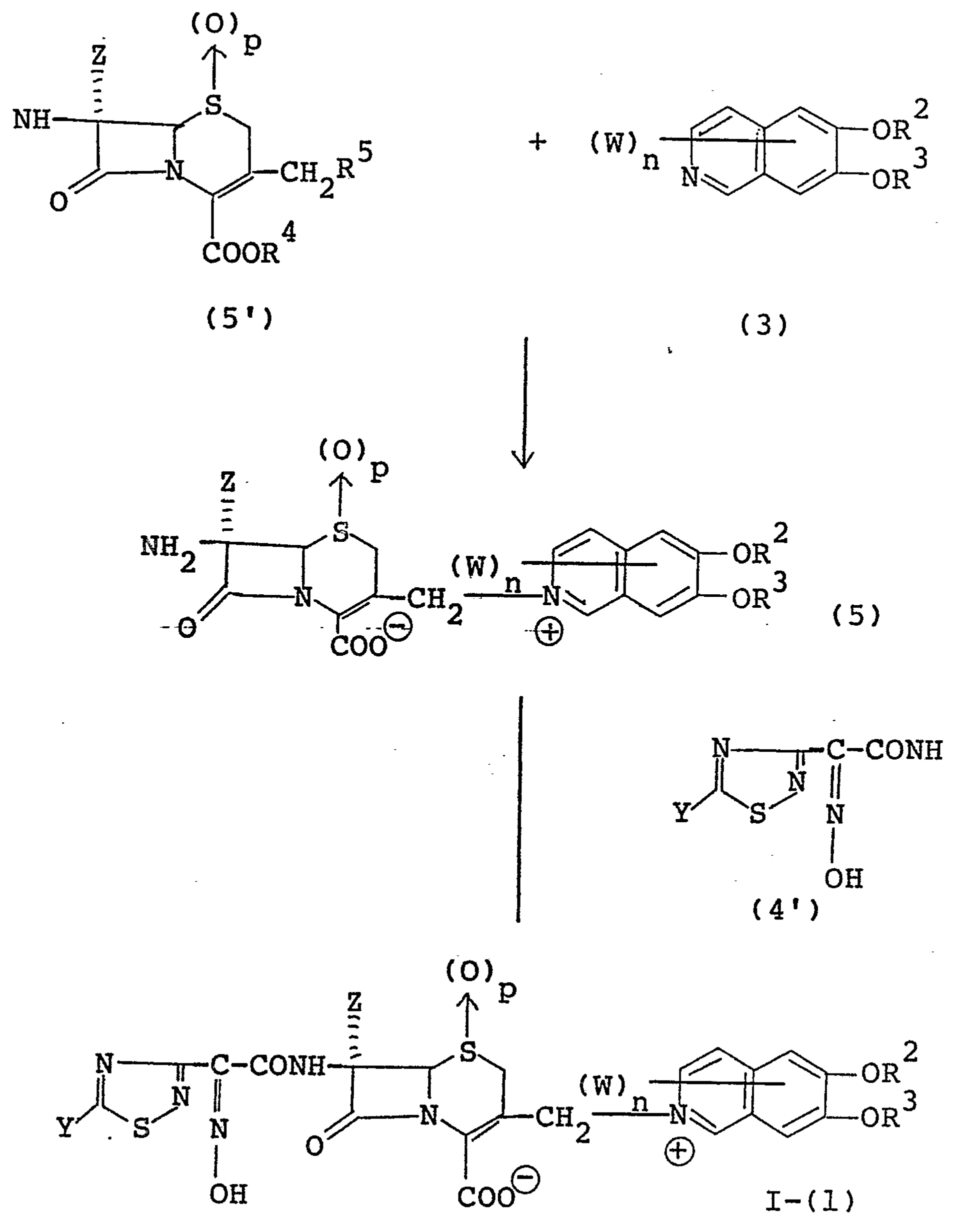

(5')

(3)

(5)

(4')

I-(1)

In the above method, the reaction of the compound of formula (5') with the compound of formula (3) can be carried out in the same way as the reaction by the reaction scheme A, and the reaction of the compound of formula (5) with the compound of formula (4') in the same way as the reaction by the reaction scheme B respectively. The compound of formula (4') is a known compound which can be formed by a known method (U.S. Patent No. 4,298,606).

In formula (10), Hal denotes a halogen atom such as chlorine, bromine or iodine. The compound of formula (10) is obtained by halogenating the corresponding aromatic hydrocarbon by a known means.

Examples of the dialkyl sulfate are dimethyl sulfate and diethyl sulfate.

Examples of the diazoalkane are diazomethane, etc.

The reaction of the compound of formula I-(1), its salt or its ester with the compound of formula (10) with a dialkyl sulfate or a diazoalkane is carried out by contacting them in an inactive solvent and if required, in the presence of a base.

Examples of the inactive solvent are alcohols such as methanol, ethanol, propanol and butanol, halogenated hydrocarbons such as methylene chloride, dichloromethane, chloroform, carbon tetrachloride and 1,2-dichloroethane, ethers such as diethyl ether, tetrahydrofuran, dioxane and dimethoxyethane, dimethylformamide, diethyl acetamide, ethyl acetate and water.

If required, the reaction may be run in the presence of a base. Examples of such base are inorganic bases such as an alkali metal hydroxide, an alkali metal bicarbonate and an alkali metal carbonate; and organic bases such as a trialkylamine, a N,N-dialkylbenzylamine, pyridine and N-alkylmorpholine. The amount of such base is usually 1 to 3 mols per mol of the compound of formula I-(1), its salt or its ester.

The amount of the compound of formula (10), the dialkyl sulfate or the diazoalkane is usually 1 to 3 mols per mol of the compound of formula I-(1), its salt or its ester.

The reaction temperature is not limited in particular; the reaction is usually conducted under cooling or under acceleration at a boiling point of a solvent.

The reaction time is usually several minutes to several tens of hours.

The reaction product can be isolated and purified by known means such as solvent extraction, crystallization and chromatography. When a syn-isomer of the compound of formula I-(1) is used as a starting compound in this reaction, a syn-isomer of the compound of formula I-(1) is obtained. When a mixture of the syn- and anti-isomers of the compound of formula I-(1) is used as a starting compound, a mixture of the syn- and anti-isomers of the compound I-(1) is obtained. Such mixture can be separated by crystallization and chromatography.

Deprotection, salt formation, esterification and reduction can be carried out in the same way as in the method by the reaction scheme A. The crystalline anhydride or hydrate of the end compound can be obtained also in the same way as shown in the method by the reaction scheme A.

In this invention, the cephalosporin compound of formula (1), above all, the cephalosporin compound of formula (1) with Y of an amino group, its pharmaceutically acceptable salt or its ester shows high antibacterial activity to wide-ranging Gram-positives and Gram-negatives and is quite useful in therapy of microbism of animals including humans. The cephalosporin compound in this invention has high antibacterial activity to Gram-negatives such as Pseudomonas aeruginosa, Salmonella typhi and Enterobacter, and Gram-positives such as Staphylococcus aureus, Streptococcus pyogenes and Bacillus subtilis. The cephalosporin derivative in this invention possesses the characteristic that it has a long duration time.

The cephalosporin compound, its pharmaceutically acceptable salt or its ester in this invention is administered either parenterally or orally. Dosage forms in case of the parenteral administration are, for example,

an intravenous or intramuscular injection and a suppository. The injection can take a form of an aqueous solution, an oily solution or a suspension. A powder and crystals used by treatment with sterilized water before administration are also available. The suppository can be a preparation obtained by containing an absorption accelerator, if required, in an ordinary excipient such as cocoa butter or glyceride.

In case of the oral administration, there are ordinary capsules, tablets and granules; they may contain an absorption accelerator and a preservative.

These various preparations can be formed by a method known per se.

The dose of the cephalosporin compound, its pharmaceutically acceptable salt or its ester varies with age and conditions of patients, and dosage forms; it is usually 10 to 2000 mg/day.

The following Examples illustrate this invention in more detail.

Example 1: (6R,7R)-7-[(Z)-2-(5-amino-1,2,4-thiadiazol-3-yl-2-methoxyimino)acetamide]-3-(6,7-dihydroxy-2-isoquinolinium)methyl-3-cephem-4-carboxylate

0.68 g of (6R,7R)-7-amino-3-iodomethyl-3-cephem-4-carboxylic acid was suspended in 20 ml of acetonitrile, and 1.51 ml of bistrimethylsilylacetamide (BSA) was stirred at room temperature for 1 hour. 0.322 g of 6,7-dihydroxyisoquinoline was suspended in 2.78 ml of DMF, 1.82 ml of BSA was added at 0°C, and stirring was conducted at room temperature for 30 minutes to form a uniform solution. Said solution was added after the above acetonitrile solution was cooled to -40°C. After the addition, the solution was stirred for 30 minutes under ice cooling. On the other hand, a substance obtained by adding 0.404 g of 5-amino-1,2,4-thiadiazol-4-yl-methoxyiminoacetic acid to 0.44 g of $PCl_5$ in 20 ml of dichloromethane at 10°C and keeping the mixture for 30 minutes was added to the above solution under ice cooling, and the resulting mixture was stirred at room temperature for 1 hour. After the reaction is over, the low-boiling solvent was distilled off under reduced pressure, and 50 ml of diethyl ether was added to the residue to obtain a brown precipitate which was separated by filtration (1.91 g of a crude product).

The crude product was extracted with 10 ml of hot water, and the extract was subjected to high-performance liquid chromatography (reversed phase ODS C-18 column, elution with a water/methanol solvent mixture) to obtain 120 mg of the captioned compound. [Compound I]

NMR (in $D_2O$ - DMSO)

3.97 (3H, S), 5.20 (1H, d), 5.60 (2H, S), 5.85 (1H, d), 7.45 (1H, S), 8.20 (2H, d+d), 9.45 (1H, S)

1R spectrum ($cm^{-1}$)

1770

Exmaple 2: (6R,7R)-7-[(Z)-5-amino-1,2,4-thiadiazol-3-yl)-2-(carboxymethoxyimino)acetamide]-3-(6,7-dihydroxy-2-isoquinolinium)methyl-3-cephem-4-carboxylate

To 5 ml of acetonitrile was suspended 0.68 g of (6R,7R)-7-amino-3-iodomethyl-3-cephem-4-carboxylic acid. Under ice cooling, 1.52 ml of BSA was added dropwise, and the mixture was stirred at room temperature for 1 hour. A solution obtained by dissolving 0.32 g of 6,7-dihydroxyisoquinoline in 3 ml of acetonitrile, adding 1.66 ml of BSA at 0°C and stirring the mixture at room temperature for 30 minutes was slowly added dropwise to the above solution at -35°C. The tempeature was gradually raised to 0°C, and the stirring continued for 30 minutes. On the other hand, 0.804 g of 5-tert-butoxycarbonylamino-1,2,4-

thiadiazol-4-yl-(tert-butoxycarbonylmethoxyimino)acetic acid was reacted with 0.50 g of $PCl_5$ in 8 ml of dichloromethane at -10°C, and the reaction mixture was further stirred at 0°C for 30 minutes. This solution was added dropwise to an acetonitrile solution at -20°C, and 0.24 ml of pyridine was further added at the same temperature. The temperature was slowly raised, and the stirring was carried out at room temperature for 1 hour. A low-boiling substance was distilled off from the reaction mixture under reduced pressure, and the residue was then dissolved in a solvent mixture of trifluoroacetic acid:-water = 10:1, and vigorous stirring was conducted at room temperature for 16 hours. The reaction mixture was poured in 500 ml of diethyl ether. The resulting precipitate was collected by filtration, and separated through a column filled with about 50 g of a CHP-20 resin with a concentration gradient of a solvent mixture of 0.1% trifluoroacetic acid containing solution and water from 100:0 to 60:40. A fraction eluted at a trifluoroacetic acid solution:HeOH = about 70:30 was collected, subjected to high-performance liquid chromatography as in Example 1 and lyophilized to obtain about 25 mg of the captioned pure compound. (Compound II)

<u>H' : NMR spectrum (in $D_6$ DMSO) $\delta$ (ppm)</u>

3.14 (2H, b), 4.62 (2H, S), 5.19 (1H, d)
5.47 (2H, b), 5.90 (1H, d+d), 7.45 (1H, S)
7.60 (1H, S), 8.16 (1H, d), 8.34 (1H, d)
9.45 (1H, S), 9.95 (1H, d)

<u>1R - spectrum ($cm^{-1}$)</u>

1770

Example 3: (6R,7R)-7-[(Z)-2-(5-amino-1,2,4-thiadiazol-3-yl)-2-(2-carboxypropyl-2-yloxyimino)acetamide]-3-(6,7-dihydroxy-2-isoquinolinium)methyl-3-ce phem-4-carboxylate

The procedure in Example 2 was followed except that 0.860 g of 5-(tert-butoxycarbonyl)amino-1,2,4-thiadiazol-3-yl-(2-tert-butoxycarbonylpropyl-2-yloxyimino)acetic acid was used instead of 0.804 of 5-(tert-butoxycarbonyl)amino-1,2,4-thiadiazol-3-yl-(tert-butoxycarbonylmethoxyimino)acetic acid. There resulted 124 mg of the captioned compound. (Compound III)

NMR spectrum (in $D_6$ DMSO) δ (ppm)

1.45 (6H, S), 3.44 (2H, b), 5.20 (1H,d)
5.47 (2H, b), 5.92 (1H, d+d), 7.44 (1H, S)
7.59 (1H, S), 8.15 (1H, d), 8.32 (1H, S)
9.49 (1H, S), 9.59 (1H, d)

Example 4: (6R,7R)-7-[(Z)-2-(5-amino-1,2,4-thiadiazol-3-yl-2-[2-carboxypropyl-2-yloxyimino)acetamide]-3-(6,7-dihydroxy-2-isoquinolinium)methyl-3-cephem-4-carboxylate

0.68 g of (6R,7R)-7-amino-3-iodomethyl-3-cephem-4-carboxylic acid was suspended in 20 ml of acetonitrile, and 1.51 ml of BSA was added, followed by stirring at room temperature for 2 hours. Subsequently a solution of 0.6 g of 6,7-di-tert-butylsiloxyisoquinoline in 5 ml of acetonitrile was added under ice cooling, and the mixture was stirred at the same temperature for 30 minutes.

Meanwhile, a solution obtained by reacting 0.860 g of 5-tert-butoxycarbonylamino-1,2,4-thiadiazol-4-yl-(2-tert-butoxycarbonylpropyl-2-yloxymino)acetic acid with 0.45 g of phosphorus pentachloride in 8 ml of dichloromethane at -10°C and further stirring the reaction mixture at room temperature for 30 minutes under ice cooling was then added to the above acetonitrile solution at -20°C. Further, 0.24 ml of pyridine was added at the same temperature. The temperature was gradually elevated to room temperature, followed by stirring for 1 hour.

To the reaction mixture was added 1 ml of methanol and the low-boiling solvent was distilled off under reduced pressure. Thereafter, the residue was dissolved in 15 ml of a solvent mixture of trifluoroacetic acid:water = 10:1, and stirred at room temperature for 24 hours. The reaction mixture was poured in 300 ml of diethyl ether, and the resulting preci pitate was collected by filtration, and separated as in Example 2 using a column filled with about 50 g of a CHP-20 resin to obtain 120 mg of the captioned compound. The NMR spectrum of the obtained compound completely agreed with that of the compound obtained in Example 3.

Example 5: (6R,7R)-7-[(Z)-2-(5-amino-1,2,4-thiadiazol-3-yl-2-[(alpha-carboxybenzyl)oxyimino]acetamide-3-(6,7-dihydroxy-2-isoquinolinium)methyl-3-cephem-4-carboxylate

1.176 g of 5-(tert-butoxycarbonyl)amino-1,2,4-thiadiazol-[(alpha-phenylmethoxycarbonyl)benzyloxyimino]-acetic acid was used instead of 0.804 g of 5-(tert-butoxycarbonyl)amino-1,2,4-thiadiazol-(tert-butoxycarbonyl-methoxyimino)acetic acid. There resulted 76 mg of the captioned compound. (Compound IV)

NMR spectrum (in $D_6$ DMSO + $D_2$ O) $\delta$ (ppm)

3.5 (2H, b), 5.14 (1H, d), 5.60 (1H, S)
5.70 (2H, S), 5.85 (1H, d), 7.4 (5H, b)
7.45 (1H, S), 7.62 (1H, S), 8.15 (1H, d)
8.25 (1H, d), 9.45 (1H, S)

Example 6: In vitro antibacterial activity

An in vitro antibacterial activity was measured by an agar disc dilution method.

Varied test bacteria were cultivated overnight in triptophane-soybroth. A Mueller Hinton agar containing the compound of this invention in varied concentrations was streaked with one platinum loopful of each of the

bacteria (number of living cells $10^6$/ml). Cultivation was conducted at 37°C for 16 hours, and minimum growth inhibitory concentration (MIC, μg/ml) was found. The results are shown in Table I.

Table I

| | I | II | III | IV | A | B | CAZ |
|---|---|---|---|---|---|---|---|
| S.aureus 209p NIHJ JC1 | 1.6 | 12.5 | 12.5 | 3.2 | 0.4 | 12.5 | 6.3 |
| S. aureus Terajima | 1.6 | 3.1 | 3.1 | 3.2 | 0.4 | 3.1 | 1.6 |
| S.pyogenes Cook | 0.05 | 0.2 | 1.6 | 0.8 | 0.2 | 1.6 | 0.4 |
| M.luteus ATCC9341 | 0.10 | 1.6 | 3.1 | 0.8 | 0.4 | 3.1 | 0.4 |
| K.pneumoniae PCI602 | 0.005 | 0.005 | 0.005 | 0.005 | 0.005 | 0.005 | 0.025 |
| S.typhi 901 | 0.005 | 0.005 | 0.005 | 0.005 | 0.005 | 0.005 | 0.1 |
| S.enteritidis G14 | 0.005 | 0.005 | 0.005 | 0.005 | 0.005 | 0.005 | 0.005 |
| S.marcescens IAM1184 | 0.005 | 0.005 | 0.005 | 0.005 | 0.005 | 0.005 | 0.025 |
| P.morganii IFO3445 | 0.005 | 0.005 | 0.005 | 0.005 | 0.1 | 0.005 | 0.0125 |

- to be continued -

Table I (continued)

| | I | II | III | IV | A | B | CAZ |
|---|---|---|---|---|---|---|---|
| P.vulgalis OX-11 | 0.005 | 0.005 | 0.005 | 0.005 | 0.005 | 0.005 | 0.005 |
| E.cloacae 963 | 0.005 | 0.005 | 0.005 | 0.005 | 0.025 | 0.005 | 0.05 |
| P.aeruginosa IFO3445 | 12.5 | 0.05 | 0.0125 | 0.025 | 50.0 | 0.025 | 0.4 |
| P.aeruginosa NCTC1049 | 0.2 | 0.0125 | 0.005 | 0.0125 | 0.2 | 0.0125 | 0.4 |
| P.aeruginosa PAD1 | 12.5 | 0.025 | 0.0125 | 0.025 | 50.0 | 0.025 | 0.4 |

A: (6R,7R)-7-[(Z)-2-(2-amino-1,3-thiazol-4-yl)-2-methoxyimino]acetamide-3-(6,7-dihydroxy-2-isoquinolinium)methyl-3-cephem-4-carboxylate

B: (6R,7R)-7-[(Z)-2-(2-amino-1,3-thiazol-4-yl)-2-(2-carboxypropyl-2-yloxyimino)acetamide]-3-(6,7-dihydroxy-2-isoquinolinium)methyl-3-cephem-4-carboxylate

CAZ: (ceftazidime)
(6R,7R)-7-[(Z)-2-(2-amino-1,3-thiazol-4-yl)-2-(2-carbcxypropyl-2-yloxyimino)acetamide]-3-(1-pyridinium)methyl-3-cephem-4-carboxylate

Comparative Example A:
(6R,7R)-7-[(Z)-2-(2-amino-1,3-thiazol-4-yl)-2-methoxyimino]acetamide-3-(6,7-dihydroxy-2-isoquinolinium)methyl-3-cephem-4-carboxylate

0.34 g of (6R,7R)-7-amino-3-iodomethyl-3-cephem-4-car= boxylic acid was suspended in 5 ml of acetonitrile, and 0.65 ml of bistrimethylsilylacetamide was added dropwise under ice cooling. Stirring was conducted for 1 hour under ice cooling to obtain a uniform mixture of trimethylsilyl (6R,7R)-7-bistrimethylsilylamino-3-iodomethyl-3-cephem-4-carboxylate. This was cooled to -20°C, and a solution of 0.161 g of 6,6-dihydroxyisoquinoline and 0.89 ml of bistrimethylsilylacetamide in 1 ml of N,N'-dimethylformamide was slowly added dropwise to provide an acetonitrile solution of (6R,7R)-7-bis-trimethylsilylamino-3-(6,7-dihydroxy-2-isoquinolinium) methyl-3-cephem-4-carboxylate.

The temperature of the obtained solution was raised to the ice cooling temperature. A solution formed separately from 0.45 g of [Z]-2-methoxyimino-2-(2-trityl-amino-1,3-thiazol-4-yl)acetic acid, 0.22 g of phosphorus pentachloride and 3 ml of $CH_2Cl_2$ with stirring for 30 minutes under ice cooling was added to the above solution. The temperature was elevated to room temperature, and the mixture was further stirred for 1 hour. Subsequently,

most of the solvent was distilled off under reduced pressure, and 3 ml of 99% formic acid was added, followed by stirring at room temperature for 2 hours. The reaction mixture was poured to 30 ml of diethyl ether, and the resulting precipitate was separated by filtration and extracted with about 10 ml of water. The extract was concentrated, and the concentrate was eluted through a CHP-20 resin column by gradually changing a water:methanol ratio from 10:1 to 7:3. A fraction eluted at a ratio of water:methanol = 8:2 was concentrated, and a main fraction was collected by high-performance chromatography (column ODS RP-18 resin) to afford 0.170 g of final (6R,7R)-7-[(Z)-2-(2-amino-1,3-thiazol-4-yl)2-methoxyiminoacetamide]-3-(6,7-dihydroxy-2-isoquinolinium)methyl-3-cephem-4-carboxylate.

[Comparative Compound A]

<u>NMR stectrum ($D_2O - d_6$ DMSO) δ(ppm):</u>

3.95 (3H, S), 5.18 (1H, d), 5.50 (1H, d), 6.74 (1H, S), 7.44 (1H, S), 7.60 (1H, S), 8.21 (2H, d+d), 9.44 (1H, S)

<u>IR spectrum ($cm^{-1}$)</u>

1778

[Comparative Example B]

(6R,7R)-7-[(Z)-2-(2-amino-1,3-thiazol-4-yl)-2-(2-carboxypropyl-2-yloxyimino)acetamide]-3-(6,7-dihydroxy-2-isoquinolinium)methyl-3-cephem-4-carboxylate

0.27 g of benzhydryl (6R,7R)-7-[(Z)-2-(2-tritylamino-1,3-thiazol-4-yl)-2-(2-tert-butoxycarbonylpropyl-2-yloxyimino)acetamide]-3-iodomethyl -3-cephem-4-carboxylate was dissolved in 25 ml of ether, and 0.081 g of 6,7-dihydroxyisoquinoline and 0.45 ml of bistrimethylsilylacetamide were added. The mixture was stirred at room temperature for 30 minutes. Further, 50 ml of ether was added to conduct dilution. The resulting precipitate

was collected by filtration, and 5 ml of 90% trifluoroacetic acid was added thereto, followed by stirring at room temperature for 2 hours. Trifluoroacetic acid was distilled off in vacuo. Ether was added to the residue. Pulverization and filtration were conducted to obtain a solid powder. To the solid powder was added 10 ml of water, and stirring was carried out for a while. Subsequently, the aqueous layer was separated and concentrated. The concentrate was collected via a CHP-20 resin column by gradually changing a water:acetone ratio from 10:1 to 6:4. A fraction eluted at a water:acetone = 8:2 was concentrated, and the concentrate was collected by high-performance chromatography and lyophilized to obtain the end compound as a 61 mg solid.

[Comparative Compound B]

NMR spectrum ($D_2O$ - d σ DMSO) δ(ppm):

1.45 (6H, S), 5.20 (1H, d), 5.55 (1H, d), 6.71 (1H, S), 7.45 (1H, S), 7.62 (1H, S), 8.23 (2H, d+d), 9.45 (1H, S)

IR spectrum ($cm^{-1}$):

1780

Example 7:

MIC values of 10 kinds of Pseudomonas aeruginosa were measured for Compound III, Comparative Example B and CAZ. The results are shown in Table II.

Table II

| | III | B | CAZ |
|---|---|---|---|
| P.aeruginosa NO. 97 | 0.005 | 0.025 | 3.1 |
| " ATCC27577 | 0.005 | 0.005 | 0.2 |
| " ATCC27318 | 0.0125 | 0.05 | 1.6 |
| " 64 | 0.0125 | 0.05 | 3.1 |
| " 88 | 0.0125 | 0.025 | 0.8 |
| " 0-11 | 0.005 | 0.05 | 3.1 |
| " N-2 | 0.025 | 0.05 | 0.8 |
| " N-3 | 0.0125 | 0.05 | 1.6 |
| " N-26 | 0.0125 | 0.1 | 1.6 |
| " Tid53 | 0.005 | 0.05 | 6.3 |

Claims

1. A cephalosporin compound represented by formula (I)

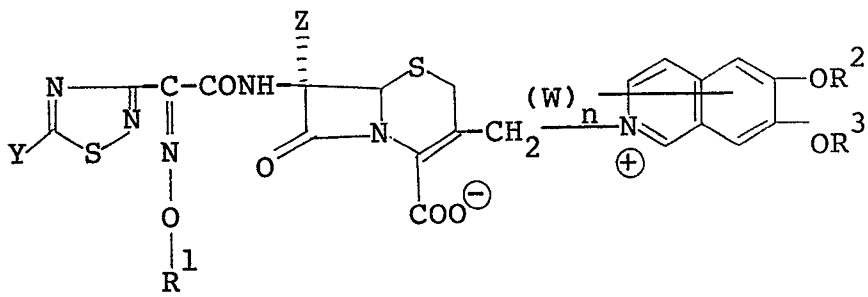

... (I)

wherein

Y denotes an amino group that may be protected,

Z denotes a hydrogen atom, a methoxy group or a formylamino group,

$R^1$ denotes an alkyl group that may be substituted or a hydrogen atom,

$R^2$ and $R^3$ are the same or different, and each denotes a hydrogen atom or a protective group of a hydroxyl group,

W denotes a substituent, and

n is an integer of 0 to 5,

or its salt.

2. A process for the production of a cephalosporin compound represented by formula (I)

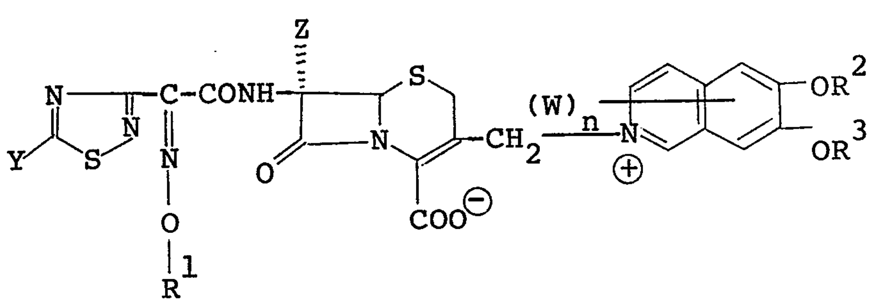

... (I)

wherein

Y denotes an amino group that may be protected,

Z denotes a hydrogen atom, a methoxy group or a formylamino group,

$R^1$ denotes an alkyl group that may be substituted or a hydrogen atom,

$R^2$ and $R^3$ are the same or different, and each denotes a hydrogen atom or a protective group of a hydroxyl group,

W denotes a substituent, and

n is an integer of 0 to 5,

or its salt, which comprises reacting a compound represented by formula (2)

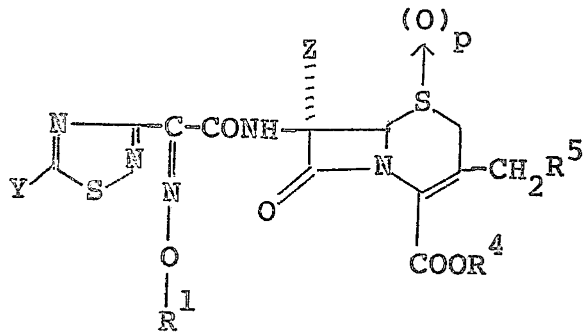

... (2)

wherein

Y, Z, $R^1$ are as defined above,

$R^5$ denotes a group that can be left by the reaction with an isoquinoline compound of formula (3) described below,

$R^4$ denotes a protective group of a carboxyl group,

and

p is 0 or 1,

with an isoquinoline compound represented by formula (3)

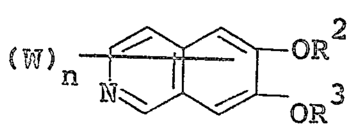

wherein $R^2$, $R^3$, W and n are as defined above, and if required, subjecting the reaction product to deprotection, reduction, esterification and salt formation.

3. A process for the production of a cephalosporin compound represented by formula (I)

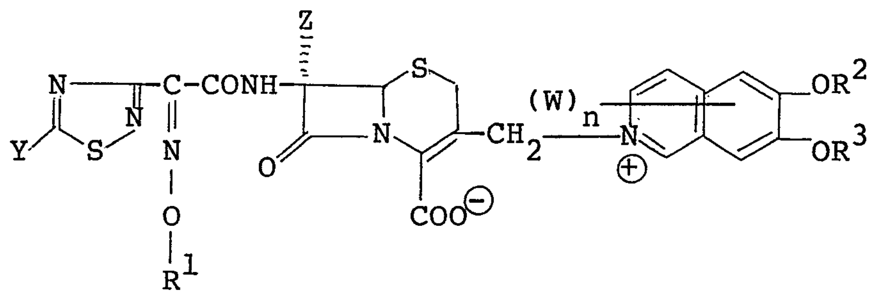 ... (I)

wherein

Y denotes an amino group that may be protected,

Z denotes a hydrogen atom, a methoxy group or a formylamino group,

$R^1$ denotes an alkyl group ~~that may be substituted~~ or a hydrogen atom,

$R^2$ and $R^3$ are the same or different, and each denotes a hydrogen atom or a protective group of a hydroxyl group,

W denotes a substituent, and

n is an integer of 0 to 5,

or its salt, which comprises reacting a compound represented by formula (4)

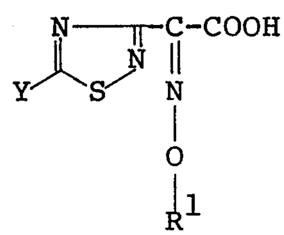 ... (4)

wherein

Y and $R^1$ are as defined above,

its salt or its reactive derivative with a compound represented by formula (5)

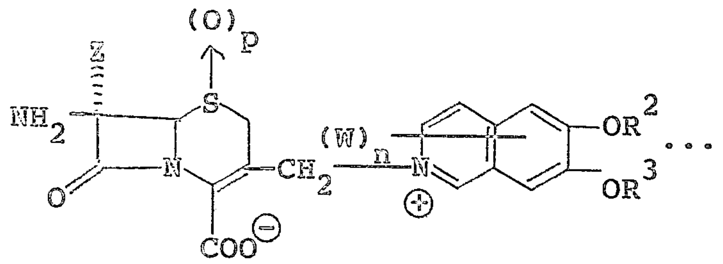
... (5)

wherein
Z, $R^2$, $R^3$, W and n are as defined above, and
p is 0 or 1,

its salt, its ester or its reactive derivative, and if required, subjecting the reaction product to deprotection, salt formation, esterification and reduction.

4. A process for the production of a cephalosporin compound represented by formula (I)

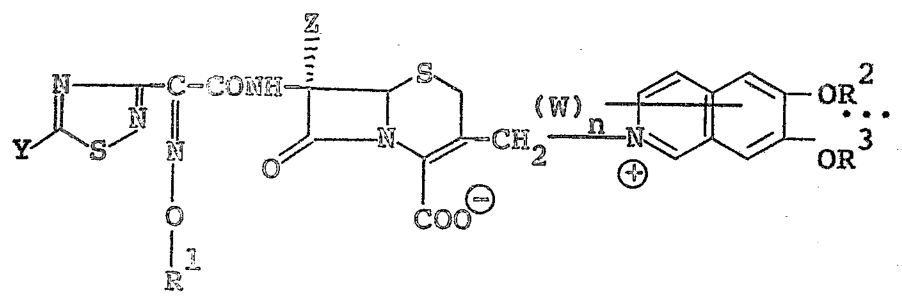
... (I)

wherein
Y denotes an amino group that may be protected,
Z denotes a hydrogen atom, a methoxy group or a formylamino group,
$R^1$ denotes an alkyl that may be substituted or a hydrogen atom,
$R^2$ and $R^3$ are the same or different, and each denotes a hydrogen atom or a protective group of a hydroxyl group,
W denotes a substituent, and
n is an integer of 0 to 5,

or its salt, which comprises reacting a compound represented by formula (6)

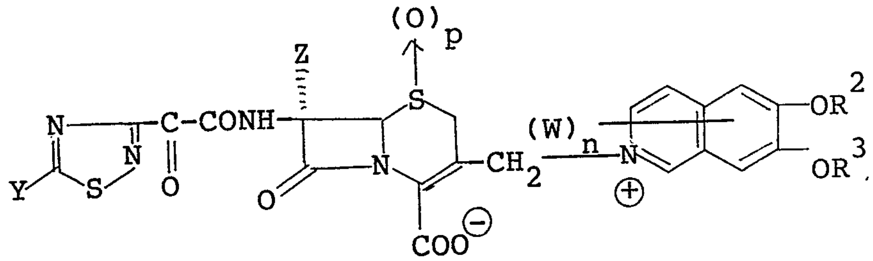 ... (6)

wherein

Y, Z, $R^2$, $R^3$, W and n are as defined above, and

p is 0 or 1,

its salt or its ester with a compound represented by formula (7)

$$NH_2\text{-}O\text{-}R^1 \quad \ldots (7)$$

wherein

$R^1$ is as defined above,

its protected compound or its salt, and if required, subjecting the reaction product to deprotection, salt formation, esterification and reduction.

5. A process for the production of a cephalosporin compound represented by formula (1)

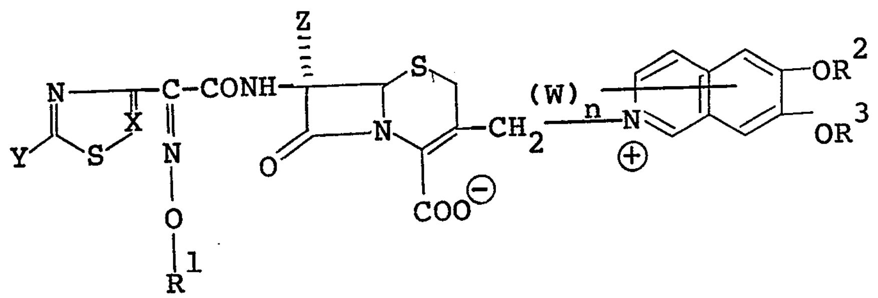 ... (I)

wherein

Y denotes an amino group that may be protected,

Z denotes a hydrogen atom, a methoxy group or a formylamino group,

$R^1$ denotes an alkyl group that may be substituted or a hydrogen atom,

$R^2$ and $R^3$ are the same or different, and each denotes a hydrogen atom or a protective group of a hydroxyl group,

W denotes a substituent, and

n is an integer of 0 to 5,

or its salt, which comprises reacting a compound represented by formula I-(1)

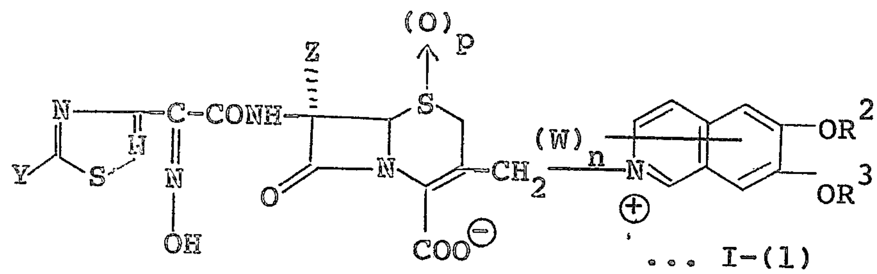

... I-(1)

wherein

Y, Z, $R^2$, $R^3$, W and n are as defined above, and

p is 0 or 1,

its salt or its ester with a compound represented by formula (10)

$$Hal - R^1 \quad ... (10)$$

wherein

$R^1$ is as defined above, and

Hal denotes a halogen atom,

and if required, subjecting the reaction product to deprotection, salt formation, esterification and reduction.

6. A pharmaceutical composition comprising an effective amount of the cephalosporin compound represented by formula (1) or its salt and a pharmaceutically acceptable inactive carrier.

# INTERNATIONAL SEARCH REPORT

International Application No PCT/JP88/00090

**I. CLASSIFICATION OF SUBJECT MATTER** (if several classification symbols apply, indicate all) [6]

According to International Patent Classification (IPC) or to both National Classification and IPC

Int.Cl$^4$ C07D501/46, 501/57, A61K31/545

**II. FIELDS SEARCHED**

Minimum Documentation Searched [7]

| Classification System | Classification Symbols |
|---|---|
| IPC | C07D501/46, 501/57, A61K31/545 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 58-57386 (Eli Lilly and Company) 5 April 1983 (05. 04. 83) Page 3, left column, line 20 to page 4, left column, line 19 & US, A, 4,396,619 & DE, A, 3,233,377 & FR, A, 2,512,448 | 1-6 |
| X | JP, A, 59-130294 (Hoechst A.G.) 26 July 1984 (26. 07. 84) Claim 3, page 7, right column, line 19 to right column, 4th line from the bottom & EP, A, 111,935 & DE, A, 3,247,614 | 1-6 |
| A | JP, A, 51-149296 (Takeda Chemical Industries, Ltd.) 8 June 1976 (08. 06. 76) Claim 3, page 7, right column, line 19 to right column, 4th line from the bottom & GB, A, 1,536,281 & DE, A, 2,556,736 | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 18, 1988 (18. 04. 88) | May 2, 1988 (02. 05. 88) |
| **International Searching Authority** | **Signature of Authorized Officer** |
| Japanese Patent Office | |